# EUROPÄISCHE PATENTANMELDUNG

(11) **EP 3 456 320 A1**
(43) Veröffentlichungstag der Anmeldung: **20.03.2019**
(21) Anmeldenummer: 17191300.7
(22) Anmeldetag: 15.09.2017
(51) Int. Cl.: A61K 9/20, A61K 31/522, A61K 31/403

(54) **DPP-4-INHIBITOR-MONO-ZUSAMMENSETZUNG**

(71) Anmelder: STADA ARZNEIMITTEL AG, 61118 Bad Vilbel (DE)
(72) Erfinder: Schönborn, Jessica, 61118 Bad Vilbel (DE)
(74) Vertreter: Kernebeck, Thomas

(57) **Zusammenfassung**

Die vorliegende Erfindung betrifft eine pharmazeutische Zusammensetzung, umfassend einen DPP-4-Inhibitor als ersten Wirkstoff sowie einen sauer reagierenden zweiten Wirkstoff. Um eine derartige Zusammensetzung bereitzustellen, die durch eine relative gute Stabilität bezüglich des DPP-4-Inhibitors gekennzeichnet ist und sich verfahrenstechnisch einfach und damit kostengünstig herstellen lässt, wird vorgeschlagen, dass der erste Wirkstoff und der zweite Wirkstoff in der Zusammensetzung in Form einer Mischung zusammen mit einem Stearat vorliegen.

## Beschreibung

Die vorliegende Erfindung betrifft eine pharmazeutische Zusammensetzung, umfassend einen DPP-4-Inhibitor als einzigen pharmazeutischen Wirkstoff, zumindest einen sauer reagierenden pharmazeutischen Hilfsstoff sowie ein Stearat.

Dipeptidylpeptidase (DPP)-4-Inhibitoren sind Substanzen, die insbesondere bei Behandlung des Diabetes mellitus Typ 2 eingesetzt werden können. Die metabolischen Wirkungen der Inhibitoren beruhen dabei auf der selektiven reversiblen kompetitiven Hemmung der Serinprotease Dipeptidylpeptidase-4 (DPP-4), die das körpereigene Inkretin GLP-1 (*Glucagon-like peptide-1*) abbaut. GLP-1, das nach einer Mahlzeit im Dünndarm in die Blutzirkulation ausgeschüttet wird, stimuliert beispielsweise die Glucose-abhängige Freisetzung von Insulin aus den Betazellen der Bauchspeicheldrüse, reduziert die Glucagonsekretion aus den Alphazellen der Bauchspeicheldrüse - und führt dadurch zu einer verminderten Glucoseproduktion in der Leber - und verlangsamt ferner die Magenentleerung in den Darm. Durch die Inhibition des Enzyms DPP-4 steigt die Konzentration des Inkretins GLP-1 an und die genannten antidiabetischen Wirkungen werden dadurch verstärkt.

DPP-4-Inhibitoren sind daher zur Behandlung von Diabetes mellitus Typ 2 zugelassen, wobei sie beispielsweise mit Biguaniden, Thiazolidindionen, Sulfonylharnstoffen und teilweise auch mit Insulin kombiniert werden können.

DPP-4-Inhibitoren an sich weisen eine verhältnismäßig hohe chemische Stabilität auf. In Formulierungen zusammen mit insbesondere sauer reagierenden pharmazeutischen Hilfsstoffen jedoch zeigen DPP-4-Inhibitoren eine nicht zu vernachlässigende Instabilität. Zur Lösung dieser Problematik schlägt WO 2009/121945 vor, einer DPP-4-Inhibitor-Formulierung ein nucleophiles und/oder basisches Agens zuzusetzen, insbesondere L-Arginin. Allerdings gestaltet es sich vergleichsweise schwierig, L-Arginin auf verfahrenstechnische einfache Weise wirkungsvoll in eine entsprechende Formulierung einzuarbeiten.

Aufgabe der vorliegenden Erfindung ist es daher, eine pharmazeutische Zusammensetzung, umfassend einen DPP-4-Inhibitor als einzigen pharmazeutischen Wirkstoff, zumindest einen sauer reagierenden pharmazeutischen Hilfsstoff sowie ein Stearat bereitzustellen, die durch eine relative gute Stabilität bezüglich des DPP-4-Inhibitors gekennzeichnet ist und sich verfahrenstechnisch einfach und damit kostengünstig herstellen lässt.

Diese Aufgabe wird gelöst durch eine pharmazeutische Zusammensetzung der eingangs genannten Art, wobei das Stearat in der Zusammensetzung mit einem Anteil von zumindest 0,75 Gew.-% enthalten ist. Überraschender Weise wurde festgestellt, dass eine Zusammensetzung, umfassend einen DPP-4-Inhibitor als einzigen pharmazeutischen Wirkstoff, zumindest einen sauer reagierenden pharmazeutischen Hilfsstoff sowie ein Stearat - sowie optional einen oder mehrere weitere, nicht sauer reagierende pharmazeutische Hilfsstoffe - eine verhältnismäßig hohe Stabilität aufweist, wenn das Stearat in der Zusammensetzung mit einem Anteil von zumindest 0,75 Gew.-% enthalten ist. Dabei kann das Stearat auf verhältnismäßig einfache Art und Weise, beispielsweise durch Untermischen des pulverförmigen Stearats, in die Zusammensetzung eingearbeitet werden, weshalb die erfindungsgemäße Zusammensetzung verhältnismäßig einfach und damit kostengünstig herstellbar ist.

Unter "Stabilität" wird in diesem Zusammenhang die von der Arbeitsgemeinschaft für pharmazeutische Verfahrenstechnik (APV) erarbeitete Definition verstanden, nach welcher "Stabilität" die spezifikationsgerechte Qualität des Arzneimittels bis zum Ende der vom Hersteller festgelegten Laufzeit bedeutet. Die Qualität des Arzneimittels wird dabei durch den Wirkstoffgehalt und die Reinheit, die sensorisch wahrnehmbaren, physikalisch-chemischen und die mikrobiologischen Eigenschaften bestimmt, wobei der Wirkstoffgehalt bis zum Ende der Laufzeit 90 % des deklarierten Wertes nicht unterschreiten soll.

Darüber hinaus weist die erfindungsgemäße Zusammensetzung eine vergleichsweise einfache Formulierung mit wenigen Komponenten auf, da Stearate in pharmazeutischen Zusammensetzungen ohnehin beispielsweise als Schmiermittel zugegen sind, was die Kosten für die Herstellung der erfindungsgemäßen Zusammensetzung weiter vermindert.

Ferner sind Stearate gängige, weitgehend inerte pharmazeutische Hilfsstoffe, bezüglich derer keine nachteiligen Inkompatibilitäten im Hinblick auf die vorliegend in Rede stehenden Wirkstoffe und im Hinblick auf andere pharmazeutische Hilfsstoffe beobachtet werden konnten.

Entsprechend einer bevorzugten Ausführungsform der erfindungsgemäßen Zusammensetzung ist es vorgesehen, dass das Stearat in der Zusammensetzung mit einem Anteil von 0,75 Gew.-% bis 10 Gew.-% enthalten ist, bevorzugt mit einem Anteil von 0,8 Gew.-% bis 5 Gew.-%, mehr bevorzugt mit einem Anteil von 0,9 Gew.-% bis 2,5 Gew.-% und noch mehr bevorzugt mit einem Anteil von 1,0 Gew.-% bis 2,5 Gew.-%. Es konnte festgestellt werden, dass wenn das Stearat in den genannten Anteilsintervallen in der erfindungsgemäßen Zusammensetzung enthalten ist, eine ausreichende Stabilität des Wirkstoffs bewerkstelligt werden kann.

Gemäß einer weiter bevorzugten Ausführungsform der erfindungsgemäßen Zusammensetzung ist es vorgesehen, dass der Wirkstoff und das Stearat in der Zusammensetzung in Form einer Mischung vorliegen. Wenn Wirkstoff und Stearat in der erfindungsgemäßen Zusammensetzung in Form einer Mischung enthalten sind, kann eine besonders hohe Stabilität des Wirkstoffs erzielt werden.

Nach einer weiter bevorzugten Ausführungsform der erfindungsgemäßen Zusammensetzung ist es vorgesehen, dass der Hilfsstoff und das Stearat in der Zusammensetzung in Form einer Mischung vorliegen. Wenn der sauer reagierende Hilfsstoff und das Stearat in der erfindungsgemäßen Zusammensetzung in Form einer Mischung enthalten sind, kann ebenfalls eine hohe Stabilität des Wirkstoffs erzielt werden.

Entsprechend einer weiter bevorzugten Ausführungsform der erfindungsgemäßen Zusammensetzung ist es vorgesehen, dass der Wirkstoff, der Hilfsstoff und das Stearat in der Zusammensetzung in Form einer innigen Mischung vorliegen. Es konnte festgestellt werden, dass der DPP-4-Inhibitor in der erfindungsgemäßen Zusammensetzung umso stabiler ist, je besser die Durchmischung von DPP-4-Inhibitor, sauer reagierendem Hilfsstoff und Stearat ist.

Gemäß einer weiter bevorzugten Ausführungsform der erfindungsgemäßen Zusammensetzung ist es vorgesehen, dass das Stearat in der Zusammensetzung gleichmäßig verteilt enthalten ist. Es wurde festgestellt, dass der DPP-4-Inhibitor umso stabiler ist, desto gleichmäßiger verteilt das Stearat in der erfindungsgemäßen Zusammensetzung enthalten ist.

Nach einer weiter bevorzugten Ausführungsform der erfindungsgemäßen Zusammensetzung kann es vorgesehen sein, dass das Stearat in der Zusammensetzung homogen verteilt enthalten ist.

Entsprechend einer weiter bevorzugten Ausführungsform der erfindungsgemäßen Zusammensetzung ist es vorgesehen, dass das Stearat ausgewählt ist aus der Gruppe bestehend aus Alkalimetall- und Erdalkalimetallstearaten und vorzugsweise aus der Gruppe bestehend aus Calcium- und Magnesiumstearat, wobei Magnesiumstearat ganz besonders bevorzugt ist. Es konnte festgestellt werden, dass die genannten Stearate, aber insbesondere Magnesiumstearat geeignet ist, den DPP-4-Inhibitor vor den Wirkungen des sauer reagierenden Hilfsstoffs hinsichtlich chemischer Degradation zu stabilisieren.

Gemäß einer weiter bevorzugten Ausführungsform der erfindungsgemäßen Zusammensetzung ist es vorgesehen, dass das Stearat in der Zusammensetzung in einer Menge von 1,0 mg bis 15 mg enthalten ist, bevorzugt in einer Menge von 1,3 mg bis 5,0 mg und mehr bevorzugt in einer Menge von 1,5 mg bis 3 mg. Es konnte festgestellt werden, dass das Stearat in den genannten Mengenbereichen den DPP-4-Inhibitor in der erfindungsgemäßen Zusammensetzung vor den Wirkungen des sauer reagierenden Hilfsstoffs hinsichtlich chemischen Abbaus ausreichend stabilisieren kann. Je nach Natur des DPP-4-Inhibitors, des sauer reagierenden Hilfsstoffs, des Stearats und möglicher weiterer pharmazeutischer Hilfsstoffe kann es vorgesehen sein, die Menge an Stearat in der Zusammensetzung auf einen Maximalwert zu begrenzen, um die Freisetzung des Wirkstoffs im beispielsweise menschlichen Körper nicht nachteilig zu beeinflussen.

In der erfindungsgemäßen Zusammensetzung ist ein DPP-4-Inhibitor als einziger Wirkstoffe enthalten. Die erfindungsgemäße Zusammensetzung umfasst demnach keinen weiteren pharmazeutischen Wirkstoff, insbesondere keinen weiteren Wirkstoff, der zur Behandlung des Diabetes mellitus geeignet ist, insbesondere nicht zur Behandlung des Diabetes mellitus Typ 2.

DPP-4-Inhibitoren, die im Stand der Technik auch als Gliptine bezeichnet werden, weisen in der Regel eine intramolekulare freie primäre oder sekundäre Aminogruppe auf. Nach einer weiter bevorzugten Ausführungsform der erfindungsgemäßen Zusammensetzung ist es daher vorgesehen, dass der DPP-4-Inhibitor eine intramolekulare freie primäre oder sekundäre Aminogruppe aufweist.

Entsprechend einer weiter bevorzugten Ausführungsform der erfindungsgemäßen Zusammensetzung ist es vorgesehen, dass der DPP-4-Inhibitor ausgewählt ist aus der Gruppe bestehend aus Alogliptin, Denagliptin, Linagliptin, Saxagliptin, Sitagliptin und Vildagliptin. Es konnte festgestellt werden, dass die genannten DPP-4-Inhibitoren in der erfindungsgemäßen Zusammensetzung vor den Wirkungen des sauer reagierenden Hilfsstoffs hinsichtlich chemischen Abbaus ausreichend stabilisiert werden können.

Die vorstehend genannten Gliptine weisen die folgenden Strukturformeln auf, wobei in den Strukturformeln die Symbole R und S (CIP-Nomenklatur) die absolute Konfiguration angeben:

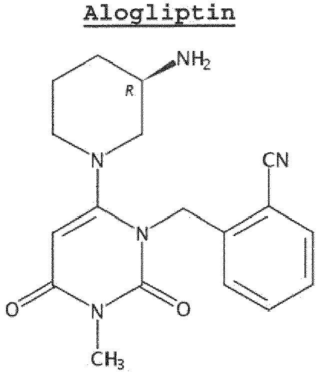

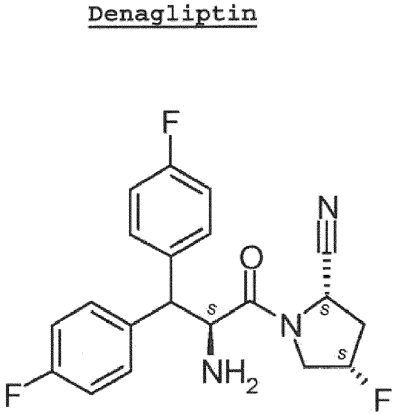

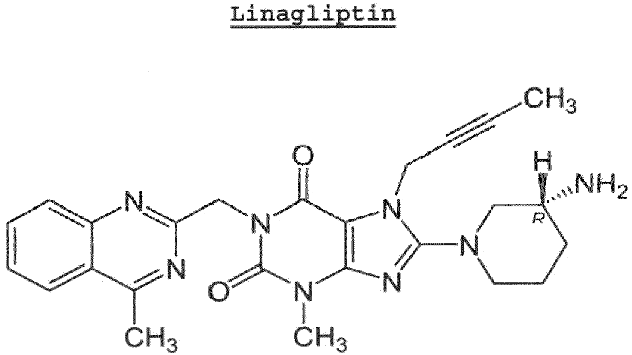

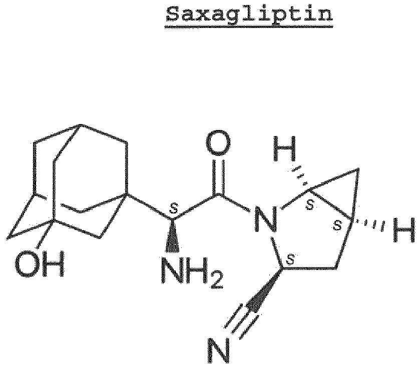

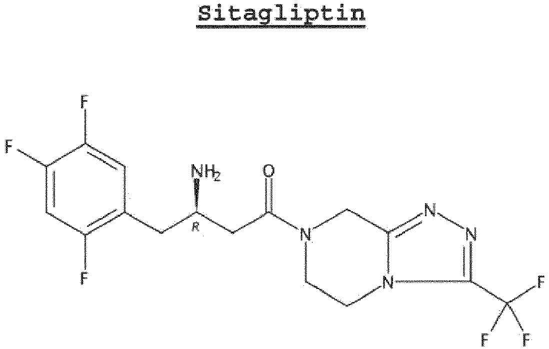

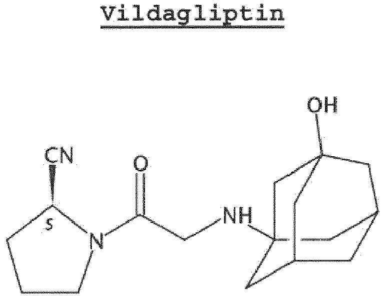

Gemäß einer weiter bevorzugten Ausführungsform der erfindungsgemäßen Zusammensetzung ist es vorgesehen, dass der DPP-4-Inhibitor Linagliptin ist.

Nach einer weiter bevorzugten Ausführungsform der erfindungsgemäßen Zusammensetzung ist es vorgesehen, dass der DPP-4-Inhibitor in der Zusammensetzung in einer Menge von 0,5 mg bis 200 mg enthalten ist, bevorzugt in einer Menge von 0,5 mg bis 100 mg und weiter bevorzugt in einer Menge von 0,5 mg, 1 mg, 2,5 mg, 5 mg, 10 mg, 50 mg oder 100 mg

Entsprechend einer weiter bevorzugten Ausführungsform der erfindungsgemäßen Zusammensetzung ist es vorgesehen, dass der DPP-4-Inhibitor in der Zusammensetzung in einer Menge von 5,0 mg enthalten ist.

Entsprechend einer weiter bevorzugten Ausführungsform der erfindungsgemäßen Zusammensetzung ist es vorgesehen, dass der Wirkstoff und das Stearat in der Zusammensetzung in einem Mengenverhältnis von 1:0,25 bis 1:10 vorliegen, vorzugsweise in einem Mengenverhältnis von 1:0,3 bis 1:3. In den genannten Mengenverhältnissen kann das Stearat den DPP-4-Inhibitor in der erfindungsgemäßen Zusammensetzung vor den Wirkungen des sauer reagierenden Hilfsstoffs hinsichtlich chemischen Abbaus signifikant stabilisieren.

In der erfindungsgemäßen Zusammensetzung ist ein sauer reagierender pharmazeutischer Hilfsstoff enthalten. Unter "sauer reagierend" wird dabei erfindungsgemäß verstanden, dass eine Lösung (bei leichtlöslichen Hilfsstoffen), eine Dispersion (bei schwerlöslichen Hilfsstoffen) bzw. eine Lösung/Dispersion (bei mäßig löslichen Hilfsstoffen) von 0,5 g des Hilfsstoffs in 10 ml destilliertem Wasser bei einer Temperatur von 25 °C einen pH-Wert von kleiner als 7,0 aufweist, bevorzugt einen pH-Wert von kleiner als 6,5 und mehr bevorzugt einen pH-Wert von kleiner als 6,0.

Entsprechend einer weiter bevorzugten Ausführungsform der erfindungsgemäßen Zusammensetzung ist es vorgesehen, dass der sauer reagierende Hilfsstoff in der Zusammensetzung in einer Menge von 0,5 mg bis 500 mg enthalten ist, vorzugsweise in einer Menge von 0,5 mg bis 200 mg und weiter bevorzugt in einer Menge von 0,8 mg bis 180 mg.

Nach einer weiter bevorzugten Ausführungsform der erfindungsgemäßen Zusammensetzung ist es vorgesehen, dass der sauer reagierende Hilfsstoff und das Stearat in der Zusammensetzung in einem Mengenverhältnis von 1:50 bis 300:1 vorliegen, vorzugsweise in einem Mengenverhältnis von 1:10 bis 200:1 und weiter bevorzugt in einem Mengenverhältnis von 1:5 bis 100:1. In den genannten Mengenverhältnissen kann das Stearat den DPP-4-Inhibitor in der erfindungsgemäßen Zusammensetzung vor den Wirkungen des sauer reagierenden Hilfsstoffs hinsichtlich chemischen Abbaus deutlich stabilisieren.

Entsprechend einer weiter bevorzugten Ausführungsform der erfindungsgemäßen Zusammensetzung ist es vorgesehen, dass die Zusammensetzung eine feste Zusammensetzung ist.

Gemäß einer weiter bevorzugten Ausführungsform der erfindungsgemäßen Zusammensetzung ist es vorgesehen, dass die Zusammensetzung eine Tablette, eine Kapsel, ein Pulver oder ein Granulat ist. Ist die erfindungsgemäße Zusammensetzung beispielsweise ein Pulver oder ein Granulat, so kann dieses erfindungsgemäß bevorzugt mittels im Stand der Technik üblicher Techniken und gegebenenfalls mittels geeigneter pharmazeutischer Hilfsstoffe zu Tabletten oder Kapseln weiterverarbeitet werden. So kann das Granulat beispielsweise mit geeigneten pharmazeutischen Hilfsstoffen vermischt und die resultierende Mischung zu einer Tablette verpresst werden. Das Granulat bildet dann die innere Phase der Tablette - oder genauer gesagt des Tablettenkerns - während die zum Granulat hinzugesetzten pharmazeutischen Hilfsstoffe die äußere Phase der Tablette/des Tablettenkerns bilden.

Nach einer weiter bevorzugten Ausführungsform der erfindungsgemäßen Zusammensetzung ist es vorgesehen, dass die Zusammensetzung eine direktverpresste Zusammensetzung ist. Direktverpresste Zusammensetzungen sind im Stand der Technik bekannt. Sie werden hergestellt, indem eine Pulvermischung ohne weitere Behandlungsschritte - wie etwa einer Granulierung - zu einem Pressling verpresst wird.

Entsprechend einer weiter bevorzugten Ausführungsform der erfindungsgemäßen Zusammensetzung ist es vorgesehen, dass der sauer reagierende pharmazeutische Hilfsstoff ausgewählt ist aus der Gruppe bestehend aus SiO₂, mikrokristalliner Cellulose und silifizierter mikrokristalliner Cellulose.

Nach einer weiter bevorzugten Ausführungsform der erfindungsgemäßen Zusammensetzung ist es vorgesehen, dass die Zusammensetzung ferner einen oder mehrere pharmazeutische Hilfsstoffe umfasst ausgewählt aus der Gruppe bestehend aus Bindemitteln, Füllmitteln, Sprengmitteln und Fließregulierungsmitteln und Schmiermitteln.

Eine typische, durch die vorliegende Erfindung bereitgestellte pharmazeutische Zusammensetzung ist ein direktverpresster Tablettenkern, umfassend 5,0 mg des DPP-4-Inhibitors Linagliptin als Wirkstoff sowie zumindest einen sauer reagierenden pharmazeutischen Hilfsstoff, wobei der Wirkstoff und der Hilfsstoff in Form einer innigen Mischung zusammen mit 1,25 mg bis 10 mg Magnesiumstearat in der Zusammensetzung vorliegen und wobei das Stearat in der Zusammensetzung mit einem Anteil von 0,75 Gew.-% bis 10 Gew.-% enthalten ist, bevorzugt mit einem Anteil von 0,8 Gew.-% bis 5 Gew.-%, mehr bevorzugt mit einem Anteil von 0,9 Gew.-% bis 2,5 Gew.-% und noch mehr bevorzugt mit einem Anteil von 1,0 Gew.-% bis 2,5 Gew.-%.

Die vorliegende Erfindung betrifft ferner ein Verfahren zur Herstellung der erfindungsgemäßen Zusammensetzung, umfassend die Schritte des
- Bereitstellens des DPP-4-Inhibitors, des sauer reagierenden pharmazeutischen Hilfsstoffs und des Stearats sowie optional zumindest eines weiteren pharmazeutischen Hilfsstoffs;
- Vermischens des DPP-4-Inhibitors, des sauer reagierenden Hilfsstoffs und des Stearates sowie gegebenenfalls des optionalen Hilfsstoffs unter Erhalt eines pulverförmigen Gemisches, wobei das Gemisch einen Anteil an dem Stearat von zumindest 0,75 Gew.-% aufweist.

Gemäß einer bevorzugten Ausführungsform des erfindungsgemäßen Verfahrens ist es vorgesehen, dass das Verfahren ferner den Schritt umfasst des
- Verpressens des pulverförmigen Gemisches unter Erhalt eines Presslings, vorzugsweise unter Erhalt einer Tablette oder eines Tablettenkerns.

Die nachfolgenden Ausführungsbeispiele dienen der Erläuterung der Erfindung.

### Ausführungsbeispiel 1:

Es wurden Filmtabletten mit der in Tabelle 1 angegebenen Formulierung hergestellt:

**Tabelle 1:**

| **1. Kerne** | | |
|---|---|---|
| | **Menge pro Tablette [mg]** | **Funktion der Substanz** |
| Linagliptin | 5,00 | Wirkstoff |
| mikrokristalline Cellulose | 157,36 | Füllmittel (sauer reagierender pharmazeutischer Hilfsstoff) |
| Hypromellose (Methocel® K100 Premium LV) | 10,80 | Bindemittel |
| Crospovidon | 3,60 | Zerfallshilfsmittel |
| hochdisperses Siliciumdioxid (Aerosil®) | 1,01 | Fließregulierungsmittel (sauer reagierender pharmazeutischer Hilfsstoff) |
| Magnesiumstearat | 2,23 | Schmiermittel und Stabilisierungsmittel zur Stabilisierung des Wirkstoffs |
| | | |
| **Gewicht Tablettenkern** | 180,00 | |

| **2. Film** | | |
|---|---|---|
| Opadry® bestehend aus: Hypromellose (E464), Talc (E553b), | 5,00 | Befilmungssystem |
| Titandioxid (E171), Macrogol (E1521), Eisenoxid rot (E 172) | | |
| **Gesamtgewicht der Filmtablette** | 185,00 | |

Die Filmtabletten wurden wie folgt hergestellt: Linagliptin, die mikrokristalline Cellulose, die Hypromellose, das Crospovidon, das hochdisperse Siliciumdioxid und das Magnesiumstearat wurden in einem konventionellen Mischer innig miteinander vermischt unter Erhalt eines pulverförmigen Gemisches. Das besagte Gemisch wurde mittels einer konventionellen Tablettenpresse zu Tablettenkernen verpresst.

Die so erhaltenen Tablettenkerne wurden in einer konventionellen Filmbeschichtungsvorrichtung durch Besprühen mit einer Beschichtungssuspension befilmt. Die Beschichtungssuspension wurde hergestellt durch Suspendieren des kommerziell erhältlichen Befilmungssystems Opadry® in Wasser.

**Stabilitätsdaten der Tabletten:**

| Lagerungsbedingungen: 40°C/75% r.h.; Packmittel: Alu/Alu - Blister | Ausgangswer [%] | 1 Monat [%] | 3 Monate [%] |
|---|---|---|---|
| Verunreinigungen (gesamt) | | | |
| | < 0. 05 | 0.07 | 0.21 |

### Ausführungsbeispiel 2:

Es wurden Filmtabletten mit der in Tabelle 2 angegebenen Formulierung hergestellt:

**Tabelle 2:**

| **1. Kerne** | | |
|---|---|---|
| | **Menge pro Tablette [mg]** | **Funktion der Substanz** |
| Linagliptin | 5,00 | Wirkstoff |
| mikrokristalline Cellulose | 157,59 | Füllmittel (sauer reagierender pharmazeutischer Hilfsstoff) |
| Hypromellose (Methocel® K100 Premium LV) | 10,80 | Bindemittel |
| Crospovidon | 3,60 | Zerfallshilfsmittel |
| hochdisperses Siliciumdioxid (Aerosil®) | 1,01 | Fließregulierungsmittel (sauer reagierender pharmazeutischer Hilfsstoff) |
| Magnesiumstearat | 2,00 | Schmiermittel und Stabilisierungsmittel zur Stabilisierung des Wirkstoffs |
| | | |
| **Gewicht Tablettenkern** | 180,00 | |

| **2. Film** | | |
|---|---|---|
| Opadry® bestehend aus: Hypromellose (E464), Talc (E553b), Titandioxid (E171), Macrogol (E1521), Eisenoxid rot (E 172) | 5,00 | Befilmungssystem |
| **Gesamtgewicht der Filmtablette** | 185,00 | |

Die Herstellung der Filmtabletten erfolgte analog Ausführungsbeispiel 1.

**Stabilitätsdaten der Tabletten:**

| Lagerungsbedingungen: 40°C/75% r.h.; Packmittel: Alu/Alu - Blister | Ausgangswer [%] | 1 Monat [%] | 3 Monate [%] |
|---|---|---|---|
| Verunreinigungen (gesamt) | | | |
| | < 0.05 | 0.09 | 0.22 |

### Ausführungsbeispiel 3:

Es wurden Filmtabletten mit der in Tabelle 3 angegebenen Formulierung hergestellt:

**Tabelle 3:**

| **1. Kerne** | | |
|---|---|---|
| | **Menge pro Tablette [mg]** | **Funktion der Substanz** |
| Linagliptin | 5,00 | Wirkstoff |
| mikrokristalline Cellulose | 157,09 | Füllmittel (sauer reagierender pharmazeutischer Hilfsstoff) |
| Hypromellose (Methocel® K100 Premium LV) | 10,80 | Bindemittel |
| Crospovidon | 3,60 | Zerfallshilfsmittel |
| hochdisperses Siliciumdioxid | 1,01 | Fließregulierungsmittel (sauer reagierender |
| (Aerosil®) | | pharmazeutischer Hilfsstoff) |
| Magnesiumstearat | 2,50 | Schmiermittel und Stabilisierungsmittel zur Stabilisierung des wirkstoffs |
| | | |
| **Gewicht Tablettenkern** | 180,00 | |

| **2. Film** | | |
|---|---|---|
| Opadry® bestehend aus: Hypromellose (E464), Talc (E553b), Titandioxid (E171), Macrogol (E1521), Eisenoxid rot (E 172) | 5,00 | Befilmungssystem |
| **Gesamtgewicht der Filmtablette** | 185,00 | |

Die Herstellung der Filmtabletten erfolgte analog Ausführungsbeispiel 1.

**Stabilitätsdaten der Tabletten:**

| Lagerungsbedingungen: 40°C/75% r.h.; Packmittel: Alu/Alu - Blister | Ausgangswert [%] | 1 Monat [%] | 3 Monate [%] |
|---|---|---|---|
| Verunreinigungen (gesamt) | | | |
| | < 0.05 | 0.07 | 0.20 |

### Ausführungsbeispiel 4:

Es wurden Filmtabletten mit der in Tabelle 4 angegebenen Formulierung hergestellt:

**Tabelle 4:**

| **1. Kerne** | | |
|---|---|---|
| | **Menge pro Tablette [mg]** | **Funktion der Substanz** |
| Saxagliptin | 5,00 | Wirkstoff |
| mikrokristalline Cellulose | 157,36 | Füllmittel (sauer reagierender pharmazeutischer Hilfsstoff) |
| Hypromellose (Methocel® K100 Premium LV) | 10,80 | Bindemittel |
| Crospovidon | 3,60 | Zerfallshilfsmittel |
| hochdisperses Siliciumdioxid (Aerosil®) | 1,01 | Fließregulierungsmittel (sauer reagierender pharmazeutischer Hilfsstoff) |
| Magnesiumstearat | 2,23 | Schmiermittel und Stabilisierungsmittel zur Stabilisierung des Wirkstoffs |
| | | |
| **Gewicht Tablettenkern** | 180,00 | |

| **2. Film** | | |
|---|---|---|
| Opadry® bestehend aus: Hypromellose (E464), Talc (E553b), Titandioxid (E171), Macrogol (E1521), Eisenoxid rot (E 172) | 5,00 | Befilmungssystem |
| **Gesamtgewicht der Filmtablette** | 185,00 | |

Die Herstellung der Filmtabletten erfolgte analog Ausführungsbeispiel 1.

### Ausführungsbeispiel 5:

Es wurden Filmtabletten mit der in Tabelle 5 angegebenen Formulierung hergestellt:

**Tabelle 5:**

| **1. Kerne** | | |
|---|---|---|
| | **Menge pro Tablette [mg]** | **Funktion der Substanz** |
| Saxagliptin | 5,00 | Wirkstoff |
| mikrokristalline Cellulose | 157,59 | Füllmittel (sauer reagierender pharmazeutischer Hilfsstoff) |
| Hypromellose (Methocel® K100 Premium LV) | 10,80 | Bindemittel |
| Crospovidon | 3,60 | Zerfallshilfsmittel |
| hochdisperses Siliciumdioxid (Aerosil®) | 1,01 | Fließregulierungsmittel (sauer reagierender pharmazeutischer Hilfsstoff) |
| Magnesiumstearat | 2,00 | Schmiermittel und Stabilisierungsmittel zur Stabilisierung des Wirkstoffs |
| | | |
| **Gewicht Tablettenkern** | 180,00 | |

| **2. Film** | | |
|---|---|---|
| Opadry® bestehend aus: Hypromellose (E464), Talc (E553b), | 5,00 | Befilmungssystem |
| Titandioxid (E171), Macrogol (E1521), Eisenoxid rot (E 172) | | |
| **Gesamtgewicht der Filmtablette** | 185,00 | |

Die Herstellung der Filmtabletten erfolgte analog Ausführungsbeispiel 1.

### Ausführungsbeispiel 6:

Es wurden Filmtabletten mit der in Tabelle 6 angegebenen Formulierung hergestellt:

**Tabelle 6:**

| **1. Kerne** | | |
|---|---|---|
| | **Menge pro Tablette [mg]** | **Funktion der Substanz** |
| Saxagliptin | 5,00 | Wirkstoff |
| mikrokristalline Cellulose | 157,09 | Füllmittel (sauer reagierender pharmazeutischer Hilfsstoff) |
| Hypromellose (Methocel® K100 Premium LV) | 10,80 | Bindemittel |
| Crospovidon | 3,60 | Zerfallshilfsmittel |
| hochdisperses Siliciumdioxid (Aerosil®) | 1,01 | Fließregulierungsmittel (sauer reagierender pharmazeutischer Hilfsstoff) |
| Magnesiumstearat | 2,50 | Schmiermittel und Stabilisierungsmittel zur Stabilisierung des |
| | | Wirkstoffs |
| | | |
| **Gewicht Tablettenkern** | 180,00 | |

| **2. Film** | | |
|---|---|---|
| Opadry® bestehend aus: Hypromellose (E464), Talc (E553b), Titandioxid (E171), Macrogol (E1521), Eisenoxid rot (E 172) | 5,00 | Befilmungssystem |
| **Gesamtgewicht der Filmtablette** | 185,00 | |

Die Herstellung der Filmtabletten erfolgte analog Ausführungsbeispiel 1.

## Patentansprüche

1. Pharmazeutische Zusammensetzung, umfassend einen DPP-4-Inhibitor als einzigen pharmazeutischen Wirkstoff, zumindest einen sauer reagierenden pharmazeutischen Hilfsstoff sowie ein Stearat, **dadurch gekennzeichnet, dass** das Stearat in der Zusammensetzung mit einem Anteil von zumindest 0,75 Gew.-% enthalten ist.

2. Zusammensetzung nach Anspruch 1, **dadurch gekennzeichnet, dass** das Stearat in der Zusammensetzung mit einem Anteil von 0,75 Gew.-% bis 10 Gew.-% enthalten ist, bevorzugt mit einem Anteil von 0,8 Gew. -% bis 5 Gew.-%, mehr bevorzugt mit einem Anteil von o,9 Gew.-% bis 2,5 Gew.-% und noch mehr bevorzugt mit einem Anteil von 1,0 Gew.-% bis 2,5 Gew.-%.

3. Zusammensetzung nach einem der voranstehenden Ansprüche, **dadurch gekennzeichnet, dass** der Wirkstoff und das Stearat in der Zusammensetzung in Form einer Mischung vorliegen.

4. Zusammensetzung nach einem der voranstehenden Ansprüche, **dadurch gekennzeichnet, dass** der Hilfsstoff und das Stearat in der Zusammensetzung in Form einer Mischung vorliegen.

5. Zusammensetzung nach einem der voranstehenden Ansprüche, **dadurch gekennzeichnet, dass** der Wirkstoff, der Hilfsstoff und das Stearat in der Zusammensetzung in Form einer innigen Mischung vorliegen.

6. Zusammensetzung nach einem der voranstehenden Ansprüche, **dadurch gekennzeichnet, dass** das Stearat Magnesiumstearat ist.

7. Zusammensetzung nach einem der voranstehenden Ansprüche, **dadurch gekennzeichnet, dass** das Stearat in der Zusammensetzung in eine Menge von 1,0 mg bis 15 mg enthalten ist, bevorzugt in einer Menge von 1,3 mg bis 5,0 mg und mehr bevorzugt in einer Menge von 1,5 mg bis 3 mg.

8. Zusammensetzung nach einem der voranstehenden Ansprüche, **dadurch gekennzeichnet, dass** der DPP-4-Inhibitor Linagliptin ist.

9. Zusammensetzung nach einem der voranstehenden Ansprüche, **dadurch gekennzeichnet, dass** der DPP-4-Inhibitor in der Zusammensetzung in einer Menge von 0,5 mg bis 200 mg enthalten ist, bevorzugt in einer Menge von 0,5 mg bis 100 mg und weiter bevorzugt in einer Menge von 0,5 mg, 1 mg, 2,5 mg, 5 mg, 10 mg, 50 mg oder 100 mg.

10. Zusammensetzung nach einem der voranstehenden Ansprüche, **dadurch gekennzeichnet, dass** der wirkstoff und das Stearat in der Zusammensetzung in einem Mengenverhältnis von 1:0,25 bis 1:10 vorliegen, vorzugsweise in einem Mengenverhältnis von 1:0,3 bis 1:3.

11. Zusammensetzung nach einem der voranstehenden Ansprüche, **dadurch gekennzeichnet, dass** die Zusammensetzung eine Tablette, eine Kapsel oder ein Granulat ist.

12. Zusammensetzung nach einem der voranstehenden Ansprüche, **dadurch gekennzeichnet, dass** die Zusammensetzung eine direktverpresste Zusammensetzung ist.

13. Zusammensetzung nach einem der voranstehenden Ansprüche, **dadurch gekennzeichnet, dass** der sauer reagierende pharmazeutische Hilfsstoff ausgewählt ist aus der Gruppe bestehend aus SiO₂, mikrokristalliner Cellulose und silifizierter mikrokristalliner Cellulose.

14. Verfahren zur Herstellung einer Zusammensetzung nach einem der voranstehenden Ansprüche, umfassend die Schritte des
- Bereitstellens des DPP-4-Inhibitors, des sauer reagierenden pharmazeutischen Hilfsstoffs und des Stearats sowie optional zumindest eines weiteren pharmazeutischen Hilfsstoffs;
- Vermischens des DPP-4-Inhibitors, des sauer reagierenden Hilfsstoffs und des Stearates sowie gegebenenfalls des optionalen Hilfsstoffs unter Erhalt eines pulverförmigen Gemisches, wobei das Gemisch einen Anteil an dem Stearat von zumindest 0,75 Gew.-% aufweist.

15. Verfahren nach Anspruch 14, ferner umfassend den Schritt des Verpressens des pulverförmigen Gemisches unter Erhalt eines Presslings, vorzugsweise unter Erhalt einer Tablette oder eines Tablettenkerns.
